# EUROPEAN PATENT SPECIFICATION

(11) **EP 2 552 200 B1**
(45) Date of publication and mention of the grant of the patent: **14.10.2015**
(21) Application number: 11763467.5
(22) Date of filing: 31.03.2011
(51) Int. Cl.: A61K 31/10, A61P 25/00

(54) **DIMETHYL SULFOXIDE (DMSO) FORMULATIONS FOR TREATING AUTISM**
DIMETHYLSULFOXID (DMSO-)FORMULIERUNGEN ZUR BEHANDLUNG VON AUTISMUS
FORMULATIONS DE DIMÉTHYLSULFOXYDE (DMSO) POUR TRAITER L'AUTISME

(30) Priority: 31.03.2010 US 319827 P
(43) Date of publication of application: 06.02.2013
(73) Proprietor: Abela Pharmaceuticals, Inc., Lake Forest, CA 92630 (US)
(72) Inventor: JACOB, Stanley, Portland, OR (US); COZEAN, Jesse, Lake Forest, CA (US); COZEAN, Colette, Lake Forest, CA (US)
(74) Representative: Rees, Kerry
(86) International application number: PCT/US2011/030806
(87) International publication number: WO 2011/123695

(56) References cited:
- WO-A1-2007/049262
- WO-A2-2008/091704
- US-A- 4 514 421
- US-A1- 2007 180 544
- US-A1- 2008 249 082
- US-A1- 2009 312 273
- US-A1- 2009 324 784
- Adams J B: "Summary of Biomedical Treatments for Autism", ARI Publication 40, 1 April 2007 (2007-04-01), pages 1-28, XP002700806, Retrieved from the Internet: URL:http://autism.com/pdf/providers/adams_ biomed_summary.pdf [retrieved on 2013-07-10]
- RAMÍREZ E ET AL: "Dimethyl sulfoxide in the treatment of mental patients.", ANNALS OF THE NEW YORK ACADEMY OF SCIENCES 15 MAR 1967, vol. 141, no. 1, 15 March 1967 (1967-03-15), pages 655-667, XP002700807, ISSN: 0077-8923
- Prater G: "Products", , 1 September 1999 (1999-09-01), pages 1-3, XP002700808, Retrieved from the Internet: URL:http://www.lef.org/magazine/mag99/sep9 9-products.htm [retrieved on 2013-07-10]

## Description

### BACKGROUND

### Field of the Invention

Embodiments of the invention relate generally to formulations comprising dimethyl sulfoxide (DMSO) to treat behavioral disorders, communication delays, and developmental delays. Several formulations disclosed herein are useful for treating broad autism phenotype disorder, including autism spectrum disorders (e.g., autism).

### Description of the Related Art

Behavioral disorders and neurodevelopmental disorders are broad categories of disorders typically used to describe the perceived behavior and developmental difficulties of children and adolescents. Such disorders are often difficult to diagnose due to their varied etiologies, differing degrees of severity, and varied times of onset.

Behavioral symptoms are used to diagnose patients and may include qualitative in social interaction, impairment in communication, restricted and/or repetitive behavior. Symptoms include lack of social or emotional reciprocity, stereotyped or repetitive use of language, idiosyncratic language, panic attacks, impaired use of nonverbal behavior, balance and coordination problems, and other generalized sensory problems.

For example, autism is a neurodevelopmental disorder that is reported to affect one of every one hundred children in the United States. It is a pervasive developmental disorder affecting social, communicative and compulsive/repetitive behaviors characterized by stereotypic complex body movements (stimming) and difficulties with normal social interaction. Children with autism may have trouble using their imagination, have a limited range of interests, and may show repetitive patterns of behavior or body movements. Autism can severely impact both the affected individual and the family members.

Once a patient is diagnosed with a behavioral and/or neurodevelopmental disorder, many current treatment regimes strive to lessen associated deficits and family distress, and to increase quality of life and functional independence of the affected individuals.

Methylsulfonylmethane (MSM; (CH3)₂SO₂₎), also known as dimethyl sulfone or methylsulfonylmethane, is an organosulfur compound that is a metabolite of DMSO and certain sulfur-containing amino acids. MSM has been marketed primarily as a dietary supplement.

Adams J B: ("Summary of Biomedical Treatments for Autism", ARI Publication 40, 1 April 2007 pages 1-28, URL:http://autism.com/pdf/providers/adams_biomed_summary.pdf) discloses p.o. MSM in a dose of 0.5 to 2 g for the treatment of autism.

Dimethyl sulfoxide (DMSO; (CH₃)₂(SO)) is a polar, aprotic solvent widely used as a solvent. It is frequently used in various chemical and biological reactions and as a cryoprotectant for cell storage. The strong unpleasant odor of DMSO, among other side effects, has adversely impacted the use of DMSO in medical applications.

RAMÍREZ E ET AL: ("Dimethyl sulfoxide in the treatment of mental patients.", ANNALS OF THE NEW YORK ACADEMY OF SCIENCES 15 MAR 1967, pages 655-667) discloses therapeutic effects of DMSO in mental patients and proposes the compound for the treatment of autism.

### SUMMARY

There is a need in the art for an efficacious treatment of neurodevelopmental disorders, such as broad autism phenotype. The phrase "broad autism phenotype", as used herein, shall be given its ordinary meaning and shall include autism spectrum disorders (such as autism, Asperger syndrome, and atypical autism), disorders characterized by personality, language and social-behavioral disorders that fall below the threshold of an autism diagnosis, and disorders having an underlying genotype associated with autism spectrum disorders.

Described herein is a formulation comprising dimethyl sulfoxide (DMSO) to treat the behavioral and physical symptoms associated with autism spectrum disorders. Alternatively, DMSO may be combined with methylsulfonylmethane (MSM) and/or other related compounds to treat the behavioral and physical symptoms associated with broad autism phenotype and other neurodevelopmental disorders, including but not limited to Attention Deficit Disorder (ADD), Attention Deficit Hyperactivity Disorder (ADHD), hyperactivity, Asperger's syndrome Tourette syndrome, Obsessive Compulsive Disorder (OCD), Pervasive Developmental Disorder (PDD), tic disorders, and learning disorders. In accordance with the invention DMSO and MSM are used to treat broad autism phenotype disorders. In several embodiments, the formulations described herein are protective and are useful for the prevention or progression of the disorders identified herein. In several embodiments, the formulations described herein act as antioxidants, aid in myelination, and/or facilitate neurotransmission.

Formulations according to several embodiments herein reduce one or more of stimming, repetitive behavior, ameliorate gastrointestinal distress, improve toilet training and/or other learned skill sets, improve language function (ability to speak as well as desire to speak), reduce social deficits, and/or improve the involuntary gag reflex suffered by many individuals having autism or other broad autism phenotype disorders.

In several embodiments, the invention comprises use of a formulation suitable for administration to a subject having a broad autism phenotype disorder for the treatment of the disorder, wherein the formulation comprises DMSO and MSM. In several embodiments, the broad autism phenotype disorder is one or more of Attention Deficit Disorder (ADD), Attention Deficit Hyperactivity Disorder (ADHD), hyperactivity, Asperger's syndrome, Tourette syndrome, Obsessive Compulsive Disorder (OCD), Pervasive Developmental Disorder (PDD), tic disorders, and learning disorders.

In several embodiments, the invention comprises a formulation comprising, consisting essentially of, or consisting of a combination of DMSO and MSM, and optionally including other ingredients. In some embodiments, DMSO is present in an amount ranging from about 0.01 g to about 5 g. In several embodiments, MSM is present in an amount ranging from about 1 g to about 10 g. In several embodiments, the formulation is configured for administration of the DMSO and the MSM individually. In several embodiments, the formulation is configured for the administration of the DMSO and the MSM together. In several embodiments, the formulation further comprises vitamins and/or dietary supplements, including but not limited to omega-3, fatty acids (such as medium chain fatty acids, lauric acid, oleic acid, coconut oil, etc.), vitamins A-E, antioxidants, amino acids, neurotransmitter precursors, herbal supplements (e.g., Lion's mane, (Hericium erinaceus) mycelium, cistanche extract; lemon balm (*Melissa officinalis*), Passiflora, St. John's wort, Chamomila), growth factors), and combinations thereof.

In some embodiments, the formulation further comprises a serotonin reuptake inhibitor. In some embodiments, the formulation further comprises risperidone. In some embodiments, the formulation further comprises urea. In some embodiments, the formulation further comprises a sulfur binding agent. In some embodiments, the formulation further comprises one or more nutrients that reduce one or more side effects associated with the components of the formulation.

In several embodiments, the formulation is in a solid form. In one embodiment, administration of the solid formulation results in timed release of the DMSO, of the MSM, of both DMSO and MSM, and/or of an optionally included additional compound. In several embodiments, the timed release of each compound is substantially similar, while in some embodiments, the solid formulation allows for individual release times of one or more components of the formulation.

In several embodiments, the MSM reduces odor associated with administration of DMSO and/or the metabolism of DMSO post-administration. In several embodiments, MSM reduces one or more additional undesired side effects caused by the DMSO. Such DMSO-related side effects include, but are not limited to gastrointestinal irritation, skin reactions (e.g., irritation at the site of administration, injection, or application), dry skin, headache, dizziness, drowsiness, nausea, vomiting, diarrhea, constipation, breathing problems, vision problems, blood problems (e.g., hemoloysis), and allergic reactions.

In several embodiments, the formulation is suitable for intravenous, sublingual, nasal, or topical administration to a subject. Other routes of administration may also be used, including but not limited to, subcutaneous injection, direct injection into a tissue site, enteric administration, epidural, intracerebral, intracerebroventricular, intradermal, intramuscular, intraperitoneal, or intravesicular. In one embodiment, the formulation is suitable for oral administration.

In several embodiments, a formulation comprising DMSO in an amount ranging from about 0.1 g to about 0.5 g is provided. In several embodiments, DMSO is present in an amount ranging from about 0.1 g to about 5 g. In several embodiments, MSM is present in an amount ranging from about 1 g to about 5 g. In several embodiments, DMSO is present in an amount ranging from about 0.005 to about 0.1 g/kg of the subject's body weight and the MSM is present in an amount ranging from about 0.1 to about 0.8 g/kg of the subject's body weight. In several embodiments, DMSO is present in a concentration of about 0.1%-23% and the MSM is present in a concentration of about 10%-25% of the total volume of the formulation. In some embodiments, ratio of MSM to DMSO is between about 20:1 to 500:1. In several embodiments wherein the formulation comprises one or more additional compounds or nutrients, the ratio of MSM and/or DMSO may be higher or lower than the range of ratios disclosed above. In some embodiments, the MSM:DMSO ratio is altered to account for the inclusion of one or more additional compounds or nutrients in the formulation. In some embodiments, however, no change in the MSM:DMSO ratio is made.

In one embodiment, the formulation is configured for a single administration to the subject for every 24 hours. In several embodiments, the formulation is configured for multiple administrations to the subject over a 24 hour period. In one embodiment, the formulation is suitable for administration between 1 and 4 times daily. In still additional embodiments, multiple formulations are provided, such that administration times can be varied based on a subject's schedule (e.g., dosing is adaptable to the patterns within the circadian rhythm of a subject having a broad autism phenotype or other neurodevelopmental disorder). For example, in some embodiments a liquid formulation is administered one or more times during the daytime to a subject, and a solid, time release formulation is administered in the early evening, such that a subject need not be woken up during their natural sleep times in order to receive an additional dose of the formulation.

In several embodiments, the formulation is suitable for co-administration with one or more additional compounds, including, but not limited to amphetamines, pemoline, methylphenidate, sertraline, atomoxetine, clomipramine, buspirone, bupropion, venlafaxine, imipramine, fluvoxamine, paroxetine, fluoxetine, nefazodone, doxepin, clozapine, haloperidol, quetiapine, thioridazine, lanzapine, carbamazepine, lithium citrate, and valproic acid. In one embodiment co-administration comprises administration of the formulation to the subject prior to administration of the one or more additional compounds to the subject. In one embodiment, co-administration comprises administration of the formulation to the subject concurrently with administration of the one or more additional compounds to the subject. In one embodiment, co-administration comprises administration of the formulation to the subject after administration of the one or more additional compounds to the subject.

In several embodiments, the formulation is suitable for the treatment of a hyperactive gag reflex. In several embodiments, the formulation is suitable for the treatment of anti-social behavior. In several embodiments, the formulation is suitable for improving language skills in a developmentally delayed subject. In several embodiments, the formulation is suitable for the treatment of stimming and/or other repetitive behavior (e.g., excessive double-checking, excessive hand-washing, anti-social outbursts, counting, tapping, repeating words, hoarding, etc.). In several embodiments, the formulation is suitable for improving social interaction in a subject. In several embodiments, the formulation is suitable for improving the ability of a subject to focus his or her attention on a task or process for an increased period of time. In some embodiments, the increased attention span further improves the social interaction, language development, or anti-social behavior of a subject.

In several embodiments, there is provided a formulation for the treatment of a broad autism phenotype disorder or symptoms associated with a broad autism phenotype disorder suitable for administration to a subject having such a disorder or such symptoms, the formulation comprising DMSO in an amount ranging from about 0.01 g to about 5 g and comprising MSM.

In one embodiment, the formulation comprises MSM, which is present in an amount ranging from about 1 g to about 10 g. In one embodiment, the formulation comprises MSM, which is present in a concentration of about 10%-25% and the DMSO is present in a concentration of about 0.1%-3% of the total volume of the formulation. In several embodiments, the formulation further comprises an additional compound or nutrient that reduces an undesired side effect of the formulation. In one embodiment, the additional compound or nutrient is a sulfur-binding agent. In one embodiment, the additional compound or nutrient is urea.

In several embodiments, the formulation is suitable for co-administration with one or more additional compounds to the subject, wherein the additional compound includes but is not limited to, amphetamines, pemoline, methylphenidate, sertraline, atomoxetine, clomipramine, buspirone, bupropion, venlafaxine, imipramine, fluvoxamine, paroxetine, fluoxetine, nefazodone, doxepin, clozapine, haloperidol, risperidone, quetiapine, thioridazine, lanzapine, carbamazepine, lithium citrate, and valproic acid.

In several embodiments, a formulation for treating broad autism phenotype disorder comprising DMSO, MSM and a serotonin reuptake inhibitor is provided. In several embodiments, a formulation for treating one or more symptoms associated with broad autism phenotype disorder comprising DMSO, MSM, and risperidone is provided. In several embodiments, the invention comprises a formulation for treating one or more symptoms associated with broad autism phenotype disorder, the formulation comprising DMSO, MSM, and one or more compounds selected from the group consisting of: amphetamine, pemoline, methylphenidate, sertraline, atomoxetine, clomipramine, buspirone, bupropion, venlafaxine, imipramine, fluvoxamine, paroxetine, fluoxetine, nefazodone, doxepin, clozapine, haloperidol, risperidone, quetiapine, thioridazine, lanzapine, carbamazepine, lithium citrate, and valproic acid. In some embodiments, the DMSO is provided in a dose of about .01 g to about 10 g, and wherein the MSM is provided in a dose of about 1 g to about 20 g.

In some embodiments, the DMSO is combined with the MSM, and wherein the combination exists as a solid form at room temperature.

In some embodiments, the DMSO and MSM is provided in a liquid formulation, wherein the liquid formulation optionally comprises a flavoring. In some embodiments, the formulation comprises a flavoring and wherein the flavoring reduces or masks an unpleasant taste associated with the formulation.

In some embodiments, the DMSO is combined with the MSM and/or an additional compound, and wherein the combination reduces an odor associated with DMSO. In one embodiment the additional compound comprises urea. In one embodiment the additional compound comprises a nutrient.

In one embodiment, the invention comprises a method for treating a broad autism phenotype disorder comprising identifying a subject having a broad autism phenotype disorder (e.g., autism), and providing DMSO and MSM to the subject. In several embodiments, the invention comprises a method for treating one or more of the following conditions: hyperactive gag reflex, stimming behavior, repetitive behavior, anti-social behavior, violent behavior, delayed language development, and delayed learned skills (e.g., toilet training) that may occur in subjects having broad autism phenotype, ADD, ADHD or other developmental and/or behavioral diagnoses. In some embodiments, the method comprises identifying a subject having said condition(s), and providing DMSO and MSM to the subject. In one embodiment, DMSO is provided in a dose of about .01 g to about 10 g, and MSM is provided in a dose of about 1 g to about 50 g. In one embodiment, DMSO is provided in a dose of about .1 g to about .5 g daily. In one embodiment, DMSO is provided in a dose of about 3 g to about 5 g daily. In one embodiment, MSM is provided in a dose of about 1 g/kg to about .8 g/kg of the subject's body weight. In one embodiment, DMSO is provided in a daily dose of .01 g to about 5 g; and MSM is provided in a dose of about 1 g to about 10 g daily. In one embodiment, DMSO is provided in a range of about .01 g to about 5 g, and MSM is provided in a range of about 1 g to about 10 g.

In one embodiment, DMSO and MSM are provided in an oral form of about 1-10 ml daily, wherein the DMSO is in a concentration of about 0.1%-2% and the MSM is provided in a concentration of about 10%-25% of the total volume. DMSO and MSM are individually provided in an oral form in one embodiment. In other embodiments, DMSO and MSM are provided in a combined oral formulation. The oral formulation is provided in a liquid form in one embodiment. Flavorings or other additives are provided in some embodiments. In some embodiments, DMSO and MSM are provided in an intravenous, sublingual, nasal, or topical form. In one embodiment, DMSO and MSM are administered topically, and the combination reduces any skin irritation that may be experienced by using DMSO alone. In one embodiment, DMSO and MSM are administered orally, and the combination reduces any gastrointestinal side effects that may be experienced by using DMSO alone.

According to some embodiments, the invention comprises a formulation or therapeutic regimen consisting or consisting essentially of DMSO and MSM for the treatment of a broad autism phenotype disorder (e.g., autism). In some embodiments, the invention comprises a therapeutic regimen comprising DMSO and MSM for the treatment of a broad autism phenotype disorder (e.g., autism), wherein said MSM and DMSO are suitable for delivery separately or combined and/or sequentially or simultaneously. In one embodiment, DMSO and MSM are delivered separately at different times (e.g., on different days or other time periods). In some embodiments, DMSO is delivered orally, and MSM is delivered topically, or vice versa.

In several of the embodiments disclosed herein, DMSO and MSM are provided in a single dose in a 24-hour period. Multiple doses in a 24-hour period are provided in another embodiment.

In several embodiments disclosed herein, DMSO and MSM are additionally provided with one or more additional compounds, including but not limited to amphetamine, pemoline, methylphenidate, sertraline, atomoxetine, clomipramine, buspirone, bupropion, venlafaxine, imipramine, fluvoxamine, paroxetine, fluoxetine, nefazodone, doxepin, clozapine, haloperidol, risperidone, quetiapine, thioridazine, lanzapine, carbamazepine, lithium citrate, and valproic acid, or combinations thereof. In one embodiment, DMSO and MSM enhances the absorption or bioavailability of the additional compound.

In several embodiments, formulations described herein are used to supplement one or more non-drug therapies, including but not limited to cognitive therapy, neuromodulation, occupational therapy, physical therapy, behavior modification, biofeedback, etc. In some embodiments, supplementing with DMSO and MSM provides a synergistic effect.

In several embodiments disclosed herein, DMSO is combined with MSM, wherein the combination exists as a solid form at room temperature. In one embodiment, the solid combination is administered to a subject with broad autism phenotype disorder or any of the symptoms associated with that disorder.

In several embodiments of the invention, a formulation for treating one or more symptoms associated with broad autism phenotype disorder is provided. In one embodiment, the formulation comprises DMSO and MSM and one or more additional compounds selected from the group consisting of: amphetamine, pemoline, methylphenidate, sertraline, atomoxetine, clomipramine, buspirone, bupropion, venlafaxine, imipramine, fluvoxamine, paroxetine, fluoxetine, nefazodone, doxepin, clozapine, haloperidol, risperidone, quetiapine, thioridazine, lanzapine, carbamazepine, lithium citrate, and valproic acid. In one embodiment, DMSO is provided in a dose of about .01 g to about 10 g, and MSM is provided in a dose of about 1 g to about 20 g. The additional compound is provided in a dose of about .25 mg to about 100 mg in some embodiments.

In one embodiment, the formulation comprises an oral dose of DMSO and MSM in solid or liquid form, and a separate dose of the additional compound. Alternatively, the formulation comprises a single dose comprising, consisting, or consisting essentially of DMSO, MSM, and at least one additional compound. In one embodiment, DMSO is combined with MSM and an additional compound, wherein the combination reduces an odor or other side effect associated with DMSO.

### DETAILED DESCRIPTION

In several embodiments, therapeutic formulations for treating broad autism phenotype disorder, are provided. In one embodiment, a formulation comprising an oral dosage of about 1-10 g/day MSM and about 0.01-2 g/day DMSO is provided. In one embodiment, a formulation comprising an oral dosage of 4.7 g/day MSM and 0.025 or 0.25 g/day DMSO is provided. The oral dosage may be in the form of a liquid solution, capsule, powder, effervescent, or any other form suitable for oral delivery.

Non-oral doses are provided according to some embodiments. In some embodiments, DMSO and MSM are provided intravenously, topically, buccally, nasally (e.g., via drops or a spray), and/or subcutaneously. Sublingual doses are provided in several embodiments. In several embodiments, the oral dosages of DMSO and MSM disclosed herein are supplemented with topical DMSO, MSM, or a combination thereof.

Broad autism phenotype (e.g., autism) includes neurodevelopmental disorders characterized by, in some cases, cerebral hypoperfusion, neuro-inflammation and gastrointestinal inflammation. In several embodiments, formulations disclosed herein are used to treat neurodevelopmental disorders. In several embodiments, formulations disclosed herein are used to treat one or more the following: cerebral hypoperfusion, neuro-inflammation and gastrointestinal inflammation. In several embodiments, formulations comprising DMSO and MSM include one or more of the following compounds: dimethyl sulfide (DMS), L-arginine, fructose 1, 6-diphosphate, L-lysine, L-aspartate, and urea. In some embodiments, formulations comprising DMSO and MSM include sulfur-binding compounds, amino acids, and/or other nutrients. In some embodiments, the additional compounds are provided in a range of percentages from about 5% to about 50% by weight of the DMSO and MSM (e.g., about 5% - 10%, 10% - 15%, 15% - 20%, 20% - 30%, 30% - 40%, 40% - 50%, and overlapping ranges thereof). Ranges of about 1% - 5% and over 50% are used in certain embodiments.

In one embodiment, the invention comprises a method of treating the symptoms of broad autism phenotype disorders, wherein the method comprises daily administration of a 10-100 ml formulation comprising 10%-15% MSM and 0.1%-10% DMSO and optional intermittent (e.g., weekly, monthly) administration of a 10-100 ml formulation comprising 10%-90% DMSO. In some embodiments, the invention comprises a method of treating the symptoms of broad autism phenotype disorders, wherein the method comprises regular administration of a 10-100 ml formulation comprising 5%-50% MSM and 0.1%-20% DMSO and optional intermittent (e.g., weekly, monthly) administration of a 10-100 ml formulation comprising 50%-100% DMSO, and optionally MSM. In some embodiments, DMSO and MSM regulate neurotransmitter activity.

In several embodiments, the combined use of MSM reduces or eliminates the odor normally associated with DMSO. This is surprisingly beneficial in several embodiments because many practitioners have avoided using DMSO in high concentrations (or in any amount) because of its unpleasant odor.

In some embodiments, a formulation comprises, consists or consists essentially of an oral dosage of about 2 g to 6 g daily MSM and about 0.01 g to 0.05 g daily of DMSO. In another embodiment, a formulation comprises, consists or consists essentially of an oral dosage of about 2 g to 6 g daily MSM and about 0.01 g to 0.2 g daily of DMSO. In another embodiment, a formulation comprises, consists or consists essentially of an oral dosage of about 2 g to 6 g daily MSM and about 0.01 g to 5.0 g daily of DMSO. In some embodiments, about 0.1 g to about 0.5 g of MSM is administered to a subject daily in, for example, single or multiple doses. In some embodiments, about 0.001 g to about 0.1 g of DMSO is administered to a subject daily in, for example, single or multiple doses. In several embodiments, dosage of MSM and DMSO is determined based on the body weight of a subject. For example, in one embodiment, the mass of MSM and DMSO administered are based on the weight of a subject in kilograms (or other unit of measure). Thus, in some embodiments, MSM is administered in a daily amount ranging from about 0.01g/kg to about 0.4 g/kg, including about 0.025 g/kg, about 0.03 g/kg, about 0.05 g/kg, about 0.1 g/kg, about 0.125 g/kg, about 0.15 g/kg, about 0.175 g/kg, about 0.20 g/kg, about 0.25 g/kg, about 0.30 g/kg, about 0.35 g/kg, and overlapping ranges thereof. Likewise, DMSO, in some embodiments, is administered in a daily amount ranging from about 0.0001 g/kg to about .2 g/kg including about 0.0002 g/kg, about 0.0003 g/kg, about 0.0005 g/kg, about 0.0007 g/kg, about 0.0009 g/kg, about 0.001 g/kg, about 0.002 g/kg, about 0.003 g/kg, about 0.004 g/kg, 0.008 g/kg, 0.012 g/kg, 0.015 g/kg, 0.02 g/kg, 0.04 g/kg, 0.06 g/kg, 0.10 g/kg, 0.12 g/kg, 0.15 g/kg, 0.175 g/kg, and overlapping ranges thereof.

In several embodiments, the invention comprises an oral formulation comprising a ratio of MSM to DMSO ranging from about 600:1 to about 40:1. In certain such embodiments, the MSM:DMSO ratio ranges from about 100:1 to about 250:1, including 120:1, 140:1, 160:1, 170:1, 180:1, 185:1, 190:1, 200:1, 220:1, and 240:1, and overlapping ranges thereof. In other embodiments, the invention comprises an oral formulation having a ratio of MSM to DMSO ranging from about 600:1 to about 5:1. Such embodiments include formulations with ratios ranging from about 10:1 to about 50:1, including 20:1, 25:1, 27.5:1, 29:1, 30:1, 31:1, 32:1, 33:1, 33.5:1, 35:1 and 40:1, and overlapping ranges thereof. In yet other embodiments, the formulation comprises a ratio of MSM to DMSO ranging from about 600:1 to 0.2:1. In such embodiments, the ratio may range from 0.2:1 to 2:1 including 0.3:1, 0.4:1, 0.5:1, 0.7:1, 0.9:1, 1.0:1, 1.1:1, 1.2:1, 1.3:1, 1.5:1, 1.7:1, and 2.0:1, and overlapping ranges thereof.

The ratio of MSM to DMSO in several embodiments is surprisingly advantageous because MSM synergistically improves the efficacy of DMSO. Thus, DMSO, which may have side effects (e.g., odor, gastrointestinal effects) when administered at certain concentrations, may be provided at lower concentrations to offer the same or better therapeutic efficacy. In some embodiments, the use of DMSO enhances the efficacy and/or lowers the amount of MSM needed. In one embodiment, DMSO reduces the side-effects and/or enhances the bioavailability (or absorbability) MSM.

In some embodiments, the invention comprises a method of managing the symptoms of neurodevelopmental disorders, such as autism, by administering an oral dosage of DMSO and MSM daily for a period of days, weeks, months or longer. In certain embodiments, a short term regimen comprises an increased daily dose of DMSO. In some embodiments, duration of use ranges from about 1 day to about 30 days (e.g., 7 days, 14 days, 20 days, etc.)

Formulations comprising DMSO and MSM are administered multiple times per day in certain embodiments. In some embodiments, dosing occurs two, three, four or more times daily, depending on age and cooperation of the patient. In other embodiments, formulations are taken only once per day.

In several embodiments, the combination of DMSO and MSM in a single formulation results in a synergistic effect, thereby lowering the concentration of DMSO, MSM, or both needed to effectively reduce one or more symptoms of autism or other neurodevelopmental disorders According to one embodiment, the use of MSM reduces the amount of DMSO needed. In one embodiment, the use of DMSO reduces the amount of MSM needed. In one embodiment, a combined DMSO and MSM therapy results in reduced concentrations of both DMSO and MSM.

Further, the synergistic effects of certain embodiments reduce the amount of time from inception of treatment to amelioration of symptoms associated with broad autism phenotype. In some embodiments, this time period is reduced several fold, for example from the order of several months down to the order of several weeks. In certain embodiments, the synergistic effects result in symptomatic improvement within 3 to 6 weeks, and in some embodiments, improvement is accomplished within days.

In several embodiments, DMSO combined with MSM is particularly advantageous because, in some embodiments, DMSO enhances the bioavailability of MSM by, for example, allowing MSM to more effectively penetrate a desired target tissue or cell. In several embodiments, DMSO is not used as a simple vehicle to enhance penetration or transportation of another agent, but rather exhibits its own bioactivity.

In several embodiments, formulations comprising DMSO and MSM exhibit reduced odor than formulations comprising DMSO alone. Formulations in liquid, gel, solid, and/or gaseous forms exhibit reduced odor when DMSO is combined with MSM in some embodiments.

As used herein, "compound" shall be given its ordinary meaning and shall include, but not be limited to, molecules, ingredients, atoms, substances and elements.

In some embodiments, the combination of DMSO and MSM unexpectedly reduces the unpleasant odor normally experienced with DMSO use. For example, in certain embodiments, DMSO/MSM formulations produce no perceptible odor after use. In some embodiments, formulations comprising DMSO and urea, and MSM are provided to reduce odor. In several embodiments in which odor is reduced, odors associated with the oxidation, metabolism, degradation or other conversion of DMSO are reduced. For example, in one embodiment, odors associated with methylthiomethane (DMS) are reduced.

In several embodiments in which odor is reduced, odors associated with the oxidation, metabolism, degradation or other conversion of DMSO are reduced. In some embodiments, odors are reduced through the binding of odiferous compounds. In some embodiments, odors are reduced by reducing the production of odor-causing compounds. In some embodiments, odors are reduced by increasing the degradation of odor-causing compounds. According to one embodiment, compounds such as MSM are provided to reduce the odor associated with DMSO use by reducing the formation of DMSO metabolites or by binding to DMSO metabolites (e.g., DMS).

In some embodiments, the odor of DMSO (or its metabolites) is reduced by about 10-20%, about 20-30%, about 30-40%, about 40-50%, about 50-75%, about 75-95%, about 95%-100%, or overlapping ranges thereof, when MSM is combined with DMSO. In one embodiment, an odor reduction of at least 50% is achieved when MSM is combined with DMSO. In one embodiment, 10% MSM reduces the odor of 90% DMSO when MSM is added to the DMSO prior to the administration of the combination to a subject. In one embodiment, MSM in a concentration of at least 5% reduces the odor of DMSO in concentrations up to 95% when MSM is added to the DMSO prior to the administration of the combination to a subject. In one embodiment, sulfur binding compounds, DMSO (or DMSO metabolite) receptor agonists or antagonists are used to reduce the odor.

Because the administration of DMSO to patients with broad autism phenotype may result in an odor that prevents treatment for patients that are attending school, the reduction of odor in several embodiments disclosed herein is particularly advantageous. In one embodiment, DMSO treatment combined with MSM can by administered daily to a patient, instead of waiting until vacations. Because of the odor-reducing properties in several embodiments, patient compliance with the formulations is increased.

In some embodiments, a coated or uncoated formulation is provided. In some embodiments, the coating provides a time-release effect (e.g., sustained-release, sustained-action, extended-release, controlled-release, or continuous-release). Coatings, in some embodiments, incorporate a flavor additive, such that there is no chalky or medicinal-tasting residue remaining after consuming the solid formulation. DMSO and MSM, may be provided in the form of oral capsules, gel-caps, tablets, powders, and effervescents, or topical gels, lotions, and creams. Other forms may also be manufactured. Liquid formulations comprising flavorings, colorings or other additives are provided in several embodiments. Confectionaries comprising DMSO and MSM are provided in some embodiments. For example, in one embodiment, a hard candy, chew or lollipop is provided to enhance the palatability of the formulation to children and/or to provide controlled release. Formulations, according to several embodiments herein, may also be incorporated into foods and beverages.

In several embodiments, a formulation comprising MSM and DMSO is encapsulated to, for example, facilitate delivery, bioactivity, absorption, time-release, and/or palatability. In several embodiments, non-encapsulated powders facilitate delivery, bioactivity, absorption, time-release, and/or palatability.

In some embodiments, one or more additional active compounds are provided with a DMSO/MSM combination. In certain embodiments, a formulation comprising DMSO and MSM further comprises vitamins and supplements, including but not limited to omega-3 fatty acids (such as medium chain fatty acids, lauric acid, oleic acid, coconut oil, etc.), vitamins A-E, antioxidants, and combinations thereof. In some embodiments, a formulation comprising DMSO and MSM further comprises stimulants, antidepressants, anti-psychotics and/or mood stabilizers (including, but not limited to, serotonin reuptake inhibitors). In some embodiments, a formulation comprising DMSO and MSM further comprises one or more of the following medications: amphetamine, pemoline, methylphenidate, sertraline, atomoxetine, clomipramine, buspirone, bupropion, venlafaxine, imipramine, fluvoxamine, paroxetine, fluoxetine, nefazodone, doxepin, clozapine, haloperidol, risperidone, quetiapine, thioridazine, lanzapine, carbamazepine, lithium citrate, and valproic acid. In some embodiments, the additional active compounds are provided in a range of percentages from about 5% to about 50% by weight of the DMSO and MSM (e.g., about 5% - 10%, 10% - 15%, 15% - 20%, 20% - 30%, 30% - 40%, 40% - 50%, and overlapping ranges thereof). Ranges of about 1% - 5% and over 50% are used in certain embodiments. In some embodiments, the ratio of MSM:DMSO by weight is 20:1 to 500:1. In some embodiments, the ratio of MSM and DMSO combined as compared to another agent (such as a medication listed above) by weight is 100:1 to 1000:1. In some embodiment, the formulation comprises about 2-10 g MSM, .01-1 g DMSO, and 2-500 mg of one or more pharmaceuticals, such as a serotonin reuptake inhibitor, risperidone, or other compound. In some embodiments, the formulation comprises about 2-10 g MSM, .01-1 g DMSO, and .5-50 g of one or more nutrients, such as a fatty acid, anti-oxidant, vitamin, etc.

In several embodiments, combination therapy of DMSO and one or more of the medications identified above (or other medications used to treat autism) is particularly advantageous because lowers doses of the medication can be used to achieve a comparable effect. Lowering the dose is particularly beneficial in some embodiments because side effects are concomitantly reduced, which may be particularly advantageous for children. In one embodiment, DMSO acts to enhance penetration of one or more of said medications (or other medications used to treat autism). In one embodiment, DMSO acts to catalyze the therapeutic reaction exerted by one or more of said medications (or other medications used to treat autism). In several embodiments, DMSO, MSM and one or more of said medications (or other medications used to treat autism) provide a synergistic effect.

In several embodiments, treatment of neurodevelopmental disorders (e.g., broad autism spectrum disorders) are accomplished only with DMSO and MSM, without the need for pharmaceuticals having undesired side-effects. In one embodiment, a formulation comprising DMSO and MSM is particularly suitable for children under the age of 6, ages at which many of the traditional pharmaceuticals are contraindicated.

In one embodiment, preventative doses of DMSO and MSM are provided to individuals who have a predisposition (e.g., biological or environmental) for neurodevelopmental disorders (e.g., broad autism spectrum disorders), but who have not yet exhibited symptoms. In some embodiments, preventative doses are in the range of about .5 g to about 5 g of MSM and about .01 g to about 0.5 g DMSO. Lower or higher ranges may be used depending on the extent of risk, age, or weight of individual.

In several embodiments, a formulation comprising DMSO and MSM is used to positively affect one or more of the following symptoms displayed by autistic individuals: stimming behavior (daytime and nighttime); gastrointestinal distress, including but not limited to severe constipation; delayed toilet training; communication function; social deficits; and hyperactive gag reflex. In several embodiments, formulations described herein improve mitochondrial function and/or facilitate oxygen transport in the body and are useful to treat broad autism phenotype, ADD, ADHD, hyperactivity and other neurodevelopmental or behavioral disorders. In several embodiments, formulations described herein reduce inflammation, including but not limited to neuro-inflammation, and are useful to treat broad autism phenotype, ADD, ADHD, hyperactivity and other neurodevelopmental or behavioral disorders.

In some embodiments, improvements in stimming are measured using the Repetitive Behavior Scales (RBS). The RBS were designed detect the presence and severity of abnormal repetitive behaviors associated with psychiatric, neurodevelopmental, and behavioral disorders. The RBS has four separate subscales, each measuring a different type of repetitive behavior: Stereotyped Behavior, Self-injurious Behavior, Compulsive Behavior, and a listing of all other aberrant behaviors. Each scale has two sections, one of which lists the observable movements, behaviors, or actions that are examples of the disorder, and a standard 7-item severity scale for each behavior. Formulations according to several embodiments disclosed herein improve at least one of the repetitive behavior types on the RBS subscales and are useful for treating psychiatric, neurodevelopmental, and behavioral disorders, including but not limited to broad autism phenotype, ADD, ADHD, hyperactivity, Asperger's syndrome, Tourette syndrome, OCD, PDD, tic disorders, and learning disorders. In some embodiments, formulations according to several embodiments herein reduce repetitive behavior by more than about 25%, 50%, 75%, 90%, 95% or 100%, in either frequency, duration, intensity, or a combination thereof.

In certain embodiments, improvements in gastrointestinal distress, including but not limited to severe constipation, are measured using the Visual-Analog Scale (VAS), which assesses the frequency and consistency of bowel movements. Formulations according to several embodiments disclosed herein improve the incidence of gastrointestinal distress, including reductions in constipation.

In certain embodiments, improvements in language function are measured using the Mullen Scales of Early Learning, testing both Expressive Language and Receptive Language. The Mullen Scales of Early Learning were designed to measure a child's early intellectual development, school readiness, and communication abilities, The Mullen Scales consist of five different ratings: Gross Motor, Fine Motor, Expressive Language, Receptive Language, and Visual Reception. Formulations according to several embodiments disclosed herein improve the performance of a patient in at least one of the Mullen Scales. In some embodiments, formulations improve a subject's sign or oral language skills, including but not limited to, learning a greater quantity of words, expediting learning, learning longer words, forming sentences, understanding context, comprehending instructions, etc. Social skills are improved in several embodiments.

In certain embodiments, improvements in social deficits are measured using the Social Responsiveness Scale (SRS). The SRS is a 65-item rating scale that measures the severity of autism spectrum symptoms as they occur in natural social settings. The SRS provides a clear picture of a patient's social impairments, assessing social awareness, social information processing, capacity for reciprocal social communication, social anxiety/avoidance, and autistic preoccupations and traits. It measures these traits in five different subsections: Receptive, Cognitive, Expressive, and Motivational aspects of social behavior, as well as Autistic Preoccupations, to provide a balanced look at the patient's improvements in social interactions. Formulations according to several embodiments disclosed herein improve the performance of a patient in one or more SRS criteria. According to some embodiments, treatment with DMSO and/or MSM, alone or combined, improve anti-social and/or violent behavior.

Formulations according to several embodiments disclosed herein reduce the severity and/or frequency of a patient's hyperactive gag reflex. In several embodiments, DMSO and MSM are used to treat patients with hyperactive gag reflex, regardless of whether neurodevelopmental symptoms are present. In some embodiments, formulations disclosed herein are used to treat disorders in oropharyngeal response. Formulations according to several embodiments herein reduce hyperactive gag reflex by more than about 25%, 50%, 75%, 90%, 95% or 100%, in either frequency, duration, intensity, or a combination thereof.

Additionally, for adolescent patients, overall improvement in autism may be measured according to the Childhood Autism Rating Scale (CARS). The CARS is used to diagnose the presence and severity of autism symptoms. The scale uses fifteen different criteria, rated 1 to 4, to objectively score a patient's behavior, interaction, and emotional responses. Use of this scale allows for the quantitative analysis of the entirety of the patient's autistic behaviors, and scores can be directly compared before and after treatment to discern the effects and benefits of DMSO/MSM treatment. Formulations according to several embodiments disclosed herein improve the patient's score in at least one of the CARS criteria.

Furthermore, qualitative measures are used to evaluate the effectiveness of DMSO/MSM therapy. Such qualitative measures include, but are not limited to general improvements autism-associated deficits, reductions family distress, increased quality of life, and increased functional independence of the affected individuals. Conclusions of improvements using the scales/scores and qualitative indices as described may be made between pre- and post-treatment evaluation of a patient, or between a post-treatment patient and an accepted average patient population with a similar degree of symptoms.

Specific embodiments will be described with reference to the following nonlimiting examples, which should be regarded in an illustrative rather than a restrictive sense.

### EXAMPLE

A male patient, diagnosed with an autism spectrum disorder at 24 months of age, was treated with a combination of MSM and DMSO, beginning at the age of 3. As described below, the patient exhibited marked improvements in the areas of stimming, gastrointestinal distress, verbalization and communication, social interaction, and hyperactive gag reflex. In some embodiments, combination of MSM and DMSO unexpectedly improve one or more symptoms associated with autism, as compared to administration of MSM or DMSO individually. In several embodiments, in addition to the beneficial therapeutic effects, the combination of MSM and DMSO reduces one or more of the side effects typically associated with DMSO administration (e.g., odor, gastrointestinal distress). In some embodiments, these unexpected effects are the result of the continued (e.g., daily) administration of MSM/DMSO, while in other embodiments, these effects are long-term (e.g., persist beyond the period of continued administration of MSM/DMSO). Moreover, in certain embodiments, combinations of MSM and DMSO result in synergistic therapeutic effects for one or more of the symptoms of autism. In some embodiments, these synergistic effects are the result of the continued (e.g., daily) administration of MSM/DMSO, while in other embodiments, these effects are long-term (e.g., persist beyond the period of continued administration of MSM/DMSO).

The patient's symptoms include stimming, nighttime stimming, hyperactive gag reflex, impaired social interaction, a lack of vocalization and communication, delayed success with toilet training, and constipation. DMSO/MSM treatment was initiated for patient. The patient was treated with 4.7 grams of oral MSM solution daily (4½ teaspoons daily of a 15% solution). Within 12 weeks, stimming (especially night-time stimming) was reduced by about 90% and incidents of hyperactive gag reflex were reduced from multiple times per day to only a few times per month. The patient was also treated with 4.7 grams of MSM and .025 grams of DMSO in an oral solution (4 ½ teaspoons daily of 15% MSM and .1% DMSO). Stimming and hyperactive gag reflex continued to improve as compared both to pre-treatment and treatment with MSM alone.

The patient was also treated with two short term bolus treatments with increased amounts of DMSO. In addition to the 4.7 g of MSM and .025 g of DMSO in an oral solution, the patient received an additional 4.8 g/day of DMSO for a short duration of 10 days (1 teaspoon, 90% oral solution). The patient's ability to vocalize improved dramatically, as did his attempts to communicate and interact. The patient's chronic constipation was also eliminated during these treatment cycles of higher doses of DMSO.

The patient was further treated with an oral formulation comprising 4.7 g of MSM and .25 g of DMSO. Improvements in communication, social interaction, and constipation continued at this dosage.

Specific examples of improvements associated with several embodiments of the invention are set forth below.

*Stimming:* The patient began exhibiting stimming behavior at 18 months of age, beginning with headshaking and spinning. At 20 months, the patient began running up and down the hall while humming in anticipation of bedtime. When put to bed, the patient would kick his crib and jump in the crib for hours at a time before falling asleep from exhaustion. Often, the patient would wake up in the middle of the night and continue jumping. After commencing treatment with a dose of 4.7 grams MSM daily (15% MSM solution, 4½ oral teaspoons daily), improvement was noted within 12 weeks. The patient was going directly to bed, lying down, and no longer kicking. His nightly stimming was reduced from every night to once every two or three weeks. Upon waking, the patient would chatter and play, rather than kick and jump. Treatment reduced the night-time stimming by about 90%, and daytime stimming by about 80%. Even upon discontinuation of MSM/DMSO administration stimming behavior, though slightly increased as compared to during treatment, was reduced compared to pre-treatment behavior. Thus, in several embodiments MSM, either alone or in combination with DMSO, reduces the incidence of stimming behavior associated with autism. In some embodiments, the combination of MSM with DMSO further reduces the incidence of stimming. In some embodiments, the combination of MSM and DMSO is particularly advantageous because higher concentrations of DMSO can be used (to achieve greater therapeutic effects) and the typical side effects of DMSO (odor, gastrointestinal effects among others) are counteracted by the MSM. In some embodiments, the methods and compositions disclosed herein are used as a chronic treatment for an autism phenotype, and/or for counteracting one or more symptoms associated therewith. In some embodiments, chronic treatment is not necessary, as the positive therapeutic effects persist beyond the period of administration.

*Social Interaction:* At 18 months of age, the patient would not engage with his parents, fighting any attention given to him. Within three months of DMSO/MSM treatment, the patient showed great social development, and became more engaging with others (e.g., giving more eye contact, and is instigating affection in the form of hugs and kisses.

When beginning school, the patient was placed in a one-on-one environment with the teacher, who felt that he was unable to participate in the routines of a regular class. After six months of DMSO/MSM treatment, the patient was introduced into a classroom with other children, where he now sits attentively and participates appropriately.

*Communication:* Before beginning DMSO/MSM therapy, the patient struggled to communicate. He knew one sign (for the word "more"). He would give a high-five when prompted, and would wave and imitate dance moves from a favorite TV show. He was non-verbal and non-vocal, with humming being his primary sound. After one month of 4.7 grams of MSM treatment daily, the patient began to engage in an activity for 8-10 minutes at a time, a marked improvement. He also gained an additional sign ("my turn") and became more vocal. Further, the patient vocalized using language-like sounds, rather than just humming.

Within about three months of DMSO/MSM treatment, the patient learned 20 sign language signs. The patient began writing out his numbers, ABCs, and his name. After continued treatment, the patient had the ability to write out his numbers from 1-20 and the entire alphabet.

After the patient was treated with a larger dose of DMSO for 10 days in addition to his normal treatment, the patient began speaking, vocalizing six different words during a session. The progress continued with daily treatment of 4.7 grams of oral MSM and .25 grams of DMSO. The patient attempted to speak more than thirty times per day and added several words to his vocabulary. Throughout treatment, his vocabulary and communicative skills increased. The patient gained a vocabulary of over 200 words, and demonstrated the ability to read post treatment. As above, these gains are maintained, even after cessation or reduction of the MSM/DMSO treatment. As discussed above, the methods and compositions disclosed herein are used as a chronic treatment for an autism phenotype, and/or for counteracting one or more symptoms associated therewith. In some embodiments, chronic treatment is not necessary, as the positive therapeutic effects persist beyond the period of administration.

*Hyperactive Gag Reflex:* Prior to DMSO/MSM treatment, the patient struggled with a hyperactive gag reflex, which manifested itself up to five times daily. At times, the reflex was so severe that the patient would vomit when witnessing someone eating, even when observing a stranger eating an ice cream cone across a shopping center. After a few months of treatment, his hyperactive gag reflex diminished dramatically. Even after cessation of therapy, the reduction in hyperactive gag reflex was maintained. Thus, while in some embodiments, the methods and compositions disclosed herein are used as a chronic treatment for an autism phenotype, and/or for counteracting one or more symptoms associated therewith, in some embodiments, chronic treatment is not necessary, as the positive therapeutic effects persist beyond the period of administration.

*Constipation:* The patient has struggled with constipation since he was a baby. This has been so severe, that he has developed a chronic anal fissure. Treatment with 90% DMSO vastly improved constipation. Improvement in constipation was also achieved with the dosage of 4.7 grams of MSM and .25 grams DMSO, with softer stool and more regular bowel movements. Upon cessation of treatment with MSM/DMSO, the patient began having difficulties with constipation. Thus in some embodiments, while other symptoms of autism can be treated on non-chronic basis (e.g., the therapeutic effects extend beyond the period of administration), maintenance doses of MSM and/or DMSO are used to maintain the positive effects on constipation. Depending on the patient, MSM and/or DMSO may be administered in response to distinct periods of constipation (e.g., non-chronic, but episodic maintenance doses). In other embodiments, a long-term maintenance dose is administered (e.g., for those patients having severe constipation issues).

While normally developing children are often fully toilet-trained by the age 2-4, many autistic children are not toilet-trained until the age of 8 years old. Even in such trained autistic children, accidents may be frequent. In some cases, the delay of toilet-training is autistic children is due to autistic children often feeling no pleasure from making their parents and teachers happy, thus causing the conventional methods that rely on a sense of pride for success with toilet training to fail. However, despite the patient's parents placing minimal focus on the issue of toilet-training or pushing the patient to complete training, after treatment with MSM/DMSO, the patient was fully toilet-trained by age 5. Thus, in some embodiments, the methods and compositions disclosed herein are useful for the treatment of a wide variety of symptoms of an autism phenotype. Moreover, in some embodiments, therapy with MSM/DMSO improves certain underlying behavioral issues present in autistic children (e.g., the psychological barriers that make toilet training difficult). In some embodiments, such improvements may be accounted for by improvements in other symptoms discussed above (e.g., toilet training is improved as a result of improved communication). However, in some embodiments, one or more of the symptoms discussed herein may be improved in the absence of one or more of the other symptoms (e.g., an autistic child may exhibit improved toilet-training in response to MSM/DMSO without a coordinate improvement in communication).

## Claims

1. A formulation for use in the treatment of a broad autism phenotype disorder, wherein the formulation comprises:
dimethylsulfoxide (DMSO); and
methylsulfonylmethane (MSM),
wherein said formulation is suitable for administration to a subject having a broad autism phenotype disorder,
wherein said DMSO is present in an amount ranging from about 0.01 g to about 10 g, and
wherein said MSM is present in an amount ranging from about 1 g to about 50 g.

2. The formulation for use according to Claim 1, wherein MSM reduces one or more undesired side effects of said DMSO, wherein said undesired side effects comprise gastrointestinal effects and/or tissue irritation, or wherein said broad autism phenotype disorder is autism.

3. The formulation for use according to Claim 1, wherein said MSM is combined with said DMSO resulting in a solid formulation at room temperature, or wherein said MSM reduces odor associated with said DMSO, thereby enhancing subject compliance in taking the formulation.

4. The formulation for use according to any one of the preceding claims, wherein said formulation further comprises one or more of a serotonin reuptake inhibitor, urea, risperidone, a sulfur binding agent, or a nutrient that reduces one or more side effects associated with the components of said formulation.

5. The formulation for use according to any of one of the preceding claims, where said formulation is suitable for the treatment of or improvement in one or more of a hyperactive gag reflex, anti-social behavior, language skills in a developmentally delayed subject, stimming and/or repetitive behavior, social interaction in a subject, or the ability of a subject to focus the subject's attention.

6. The formulation for use according to any of one of the preceding claims, wherein said formulation is configured to administer said DMSO and said MSM individually.

7. The formulation for use according to any of claims, 1-5 wherein said formulation is configured to administer said DMSO and said MSM in combination.

8. The formulation for use according to any of one of Claims 1 and 3-7, wherein said MSM reduces odor associated with said DMSO or reduces one or more undesired side effects caused by said DMSO.

9. The formulation for use according to Claim 8, wherein said side effects comprise gastrointestinal effects and/or tissue irritation.

10. The formulation for use according to any one of the preceding claims, wherein said DMSO is present in an amount ranging from about 0.01 g to about 5 g, and wherein said MSM is present in an amount ranging from about 1 g to about 5 g.

11. The formulation for use according to any one of Claims 1-10, wherein said DMSO is present in an amount ranging from about 0.005 to about 0.1 g/kg of said subject's body weight and said MSM is present in an amount ranging from about 0.1 to about 0.8 g/kg of said subject's body weight.

12. The formulation for use according to any one of Claims 1-10, wherein said DMSO is present in a concentration of about 0.1%-3% and said MSM is present in a concentration of about 10%-25% of the total volume of the formulation.

13. The formulation for use according to any one of Claims 1-10, wherein the ratio of MSM to said DMSO is between about 20:1 to 500:1.

14. The formulation for use according to any one of the preceding claims, wherein said formulation is configured for administration between 1 and 4 times daily.

15. The formulation for use according to any one of the preceding claims, wherein said formulation is in a solid form and wherein administration of said solid formulation results in timed release of said DMSO and said MSM.

16. An oral formulation for use in the treatment of a broad autism phenotype disorder, wherein the formulation comprises:
dimethylsulfoxide (DMSO); and
methylsulfonylmethane (MSM),
wherein said formulation is suitable for administration to a subject having a broad autism phenotype disorder.

17. A formulation for use in the treatment of a broad autism phenotype disorder, wherein the formulation comprises:
dimethylsulfoxide (DMSO); and
methylsulfonylmethane (MSM),
wherein said formulation is suitable for administration to a subject having a broad autism phenotype disorder.

## Patentansprüche

1. Formulierung für die Verwendung bei der Behandlung einer breiten Autismus-Phänotyp-Störung, worin die Formulierung Folgendes umfasst:
Dimethylsulfoxid (DMSO); und
Methylsulfonylmethan (MSM),
worin die genannte Formulierung für die Verabreichung an ein Subjekt mit einer breiten Autismus-Phänotyp-Störung geeignet ist,
worin das genannte DMSO in einer Menge im Bereich von etwa 0,01 g bis etwa 10 g vorliegt, und
worin das genannte MSM in einer Menge im Bereich von etwa 1 g bis etwa 50 g vorliegt.

2. Formulierung für die Verwendung nach Anspruch 1, worin MSM eine oder mehrere unerwünschte Nebenwirkungen des genannten DMSO verringert, worin die genannten unerwünschten Nebenwirkungen Wirkungen auf den Magen-Dram-Trakt und/oder Gewebereizung umfassen oder worin die genannte breite Autismus-Phänotyp-Störung Autismus ist.

3. Formulierung für die Verwendung nach Anspruch 1, worin das genannte MSM mit dem genannten DMSO kombiniert ist, was zu einer festen Formulierung bei Raumtemperatur führt, oder worin das genannte MSM den Geruch reduziert, der mit dem genannten DMSO assoziiert wird, wodurch die Compliance des Subjekts zur Einnahme der Formulierung verbessert wird.

4. Formulierung für die Verwendung nach einem der vorstehenden Ansprüche, worin die genannte Formulierung weiter eines oder mehrere der Folgenden umfasst: einen Serotonin-Wiederaufnahme-Hemmer, Harnstoff, Risperidon, ein Schwefelbindendes Mittel oder einen Nährstoff, der eine oder mehrere Nebenwirkungen, die mit den Komponenten der genannten Formulierung assoziiert werden, verringert.

5. Formulierung für die Verwendung nach einem der vorstehenden Ansprüche, worin die genannte Formulierung für die Behandlung oder Verbesserung von einem oder mehreren der Folgenden geeignet ist: einem hyperaktiven Würgreflex, antisozialem Verhalten, Sprachfähigkeiten bei einem entwicklungsverzögerten Subjekt, Stimming und/oder repetitivem Verhalten, sozialer Interaktion bei einem Subjekt oder der Fähigkeit eines Subjekts, die Aufmerksamkeit des Subjekts zu fokussieren.

6. Formulierung für die Verwendung nach einem der vorstehenden Ansprüche, worin die genannte Formulierung zur einzelnen Verabreichung des genannten DMSO und des genannten MSM konfiguriert ist.

7. Formulierung für die Verwendung nach einem der Ansprüche 1-5, worin die genannte Formulierung zur kombinierten Verabreichung des genannten DMSO und des genannten MSM konfiguriert ist.

8. Formulierung für die Verwendung nach einem der Ansprüche 1 und 3-7, worin das genannte MSM den Geruch reduziert, der mit dem genannten DMSO assoziiert wird, oder eine oder mehrere unerwünschte Nebenwirkungen, die durch das genannte DMSO verursacht werden, verringert.

9. Formulierung für die Verwendung nach Anspruch 8, worin die genannten Nebenwirkungen Wirkungen auf den Magen-Dram-Trakt und/oder Gewebereizung umfassen.

10. Formulierung für die Verwendung nach einem der vorstehenden Ansprüche, worin das genannte DMSO in einer Menge im Bereich von etwa 0,01 g bis etwa 5 g vorliegt und worin das genannte MSM in einer Menge im Bereich von etwa 1 g bis etwa 5 g vorliegt.

11. Formulierung für die Verwendung nach einem der Ansprüche 1-10, worin das genannte DMSO in einer Menge im Bereich von etwa 0,005 bis etwa 0,1 g/kg des Körpergewichts des genannten Subjekts vorliegt und das genannte MSM in einer Menge im Bereich von etwa 0,1 bis etwa 0,8 g/kg des Körpergewichts des genannten Subjekts vorliegt.

12. Formulierung für die Verwendung nach einem der Ansprüche 1-10, worin das genannte DMSO in einer Konzentration von etwa 0,1 %-3 % vorliegt und das genannte MSM in einer Konzentration von etwa 10 %-25 % des Gesamtvolumens der Formulierung vorliegt.

13. Formulierung für die Verwendung nach einem der Ansprüche 1-10, worin das Verhältnis von MSM zu dem genannten DMSO zwischen etwa 20 : 1 bis 500 : 1 beträgt.

14. Formulierung für die Verwendung nach einem der vorstehenden Ansprüche, worin die genannte Formulierung zur Verabreichung zwischen 1- und 4-mal täglich konfiguriert ist.

15. Formulierung für die Verwendung nach einem der vorstehenden Ansprüche, worin die genannte Formulierung in einer festen Form vorliegt und worin die Verabreichung der genannten festen Formulierung zu einer zeitversetzten Freisetzung des genannten DMSO und des genannten MSM führt.

16. Orale Formulierung für die Verwendung bei der Behandlung einer breiten Autismus-Phänotyp-Störung, worin die Formulierung Folgendes umfasst:
Dimethylsulfoxid (DMSO); und
Methylsulfonylmethan (MSM),
worin die genannte Formulierung für die Verabreichung an ein Subjekt mit einer breiten Autismus-Phänotyp-Störung geeignet ist.

17. Formulierung für die Verwendung bei der Behandlung einer breiten Autismus-Phänotyp-Störung, worin die Formulierung Folgendes umfasst:
Dimethylsulfoxid (DMSO); und
Methylsulfonylmethan (MSM),
worin die genannte Formulierung für die Verabreichung an ein Subjekt mit einer breiten Autismus-Phänotyp-Störung geeignet ist.

## Revendications

1. Formulation pour une utilisation dans le traitement de troubles caractéristiques d'un phénotype élargi de l'autisme, la formulation comprenant :
du diméthylsulfoxyde (DMSO) ; et
du méthylsulfonylméthane (MSM),
ladite formulation se prêtant à une administration chez un sujet ayant des troubles caractéristiques d'un phénotype élargi de l'autisme,
dans laquelle ledit DMSO est présent à une quantité qui va d'environ 0,01 g à environ 10 g et
dans laquelle ledit MSM est présent à une quantité qui va d'environ 1 g à environ 50 g.

2. Formulation pour l'utilisation selon la revendication 1, dans laquelle le MSM amoindrit un ou plusieurs des effets secondaires indésirables dudit DMSO, lesdits effets secondaires indésirables comprenant des effets gastro-intestinaux et/ou une irritation tissulaire ou lesdits troubles caractéristiques d'un phénotype élargi de l'autisme étant un autisme.

3. Formulation pour l'utilisation selon la revendication 1, dans laquelle ledit MSM est combiné avec ledit DMSO pour obtenir une formulation solide à la température ambiante ou dans laquelle ledit MSM réduit l'odeur associée au dit DMSO, augmentant ainsi l'adhésion du sujet au traitement par la formulation.

4. Formulation pour l'utilisation selon l'une quelconque des revendications précédentes, ladite formulation comprenant en outre un ou plusieurs des ingrédients suivants : inhibiteur du recaptage de la sérotonine, urée, rispéridone, agent de fixation du soufre ou nutriment qui amoindrit un ou plusieurs des effets secondaires liés aux composants de ladite formulation.

5. Formulation pour l'utilisation selon l'une quelconque des revendications précédentes, ladite formulation se prêtant au traitement ou à l'amélioration d'un ou de plusieurs des aspects suivants : hyperactivité du réflexe pharyngé, comportement antisocial, compétences linguistiques chez un sujet qui présente un retard développemental, comportement auto-stimulatoire et/ou répétitif, interactions sociales du sujet ou capacité du sujet à concentrer son attention.

6. Formulation pour l'utilisation selon l'une quelconque des revendications précédentes, ladite formulation étant configurée pour administrer ledit DMSO et ledit MSM individuellement.

7. Formulation pour l'utilisation selon l'une quelconque des revendications 1-5, ladite formulation étant configurée pour administrer ledit DMSO et ledit MSM en combinaison.

8. Formulation pour l'utilisation selon l'une quelconque des revendications 1 et 3-7, dans laquelle ledit MSM réduit l'odeur associée au dit DMSO ou amoindrit un ou plusieurs des effets indésirables causés par ledit DMSO.

9. Formulation pour l'utilisation selon la revendication 8, lesdits effets secondaires comprenant des effets gastro-intestinaux et/ou une irritation tissulaire.

10. Formulation pour l'utilisation selon l'une quelconque des revendications précédentes, dans laquelle ledit DMSO est présent à une quantité qui va d'environ 0,01 g à environ 5 g et dans laquelle ledit MSM est présent à une quantité qui va d'environ 1 g à environ 5 g.

11. Formulation pour l'utilisation selon l'une quelconque des revendications 1-10, dans laquelle ledit DMSO est présent à une quantité qui va d'environ 0,005 à environ 0,1 g/kg de poids corporel dudit sujet et dans laquelle ledit MSM est présent à une quantité qui va d'environ 0,1 à environ 0,8 g/kg de poids corporel dudit sujet.

12. Formulation pour l'utilisation selon l'une quelconque des revendications 1-10, dans laquelle ledit DMSO est présent à une concentration d'environ 0,1 %-3 % et ledit MSM est présent à une concentration d'environ 10 %-25 % du volume total de la formulation.

13. Formulation pour l'utilisation selon l'une quelconque des revendications 1-10, dans laquelle le rapport entre le MSM et ledit DMSO est entre environ 20:1 à 500:1.

14. Formulation pour l'utilisation selon l'une quelconque des revendications précédentes, ladite formulation étant configurée pour être administrée entre 1 et 4 fois par jour.

15. Formulation pour l'utilisation selon l'une quelconque des revendications précédentes, ladite formulation étant sous une forme solide et l'administration de ladite formulation solide résultant en une libération échelonnée dudit DMSO et dudit MSM.

16. Formulation orale pour une utilisation dans le traitement de troubles caractéristiques d'un phénotype élargi de l'autisme, la formulation comprenant :
du diméthylsulfoxyde (DMSO) ; et
du méthylsulfonylméthane (MSM),
ladite formulation se prêtant à une administration chez un sujet ayant des troubles caractéristiques d'un phénotype élargi de l'autisme.

17. Formulation pour une utilisation dans le traitement de troubles caractéristiques d'un phénotype élargi de l'autisme, la formulation comprenant :
du diméthylsulfoxyde (DMSO) ; et
du méthylsulfonylméthane (MSM),
ladite formulation se prêtant à une administration chez un sujet ayant des troubles caractéristiques d'un phénotype élargi de l'autisme.
